# EUROPEAN PATENT SPECIFICATION

(11) **EP 0 501 056 B1**
(45) Date of publication and mention of the grant of the patent: **19.06.1996**
(21) Application number: 91303824.6
(22) Date of filing: 26.04.1991
(51) Int. Cl.: A61K 31/58, A61K 9/02

(54) **Danazol containing compositions for the treatment of endometriosis**
Danazol enthaltende Arzneimittel zur Behandlung von Endometriosis
Compositions à base de danazole pour le traitement de l'endométriose

(30) Priority: 28.02.1991 JP 34307/91
(43) Date of publication of application: 02.09.1992
(73) Proprietor: Igarashi, Masao, Maebashi-shi Gunma-Ken (JP)
(72) Inventor: Igarashi, Masao, Maebashi-shi Gunma-Ken (JP)
(74) Representative: Coxon, Philip

(56) References cited:
- EP-A- 0 330 786
- STN INTERNATIONAL INFORMATION SERVICES, DATA BASE: CHEMICAL ABSTRACTS, vol. 115, no. 16, abstract no. 166673q, Columbus, Ohio, US; & JP-A-03 090 029

## Description

### BACKGROUND OF THE INVENTION

### Field of the art

The present invention relates to a therapeutic agent of endometriosis for topical dosage. More specifically, the present invention relates to a therapeutic agent of endometriosis for parenteral non-vaginal administration comprising danazol as an effective component.

### Related art

Endometriosis is defined as the condition in which tissue resembling endometrium is found in various extrauterine locations, chiefly in the pelvic cavity, as described in Novak's Textbook of Gynecology. The endometriosis found in the pelvic cavity is called as pelvic endometriosis. Adenomyosis of the uterus is characterized by histologically benign invasion of the uterine musculature (= myometrium) by the endometriosis. In both pelvic endometriosis and adenomyosis, there is ectopic growth of endometrial tissue. For that matter, adenomyosis was at one time spoken of as endometriosis interna, to distinguish it from endometriosis externa or pelvic endometriosis. Thus the term "endometriosis" is herein referred to as including both pelvic endometriosis and adenomyosis.

An increased number of women have suffered from infertility or dysmenorrhea due to pelvic endometriosis or adenomyosis uteri.

Danazol is known as a drug for the oral treatment of pelvic endometriosis.

Danazol is a drug comprising 17α-pregna-2,4-dien-20-yno[2,3-d]-isoxazol-17-ol as its chemical entity, and it is believed that danazol, which acts as an inhibitor at the anterior lobe of pituitary (Stedman's Medical Dictionary), inhibits the secretion of hormones from the hypothalamus or pituitary or from ovary upon its oral administration and thereby exhibits therapeutic effect on endometriosis. Thus, danazol having such a functional mechanism as above has been used as a systemic therapeutic agent for oral administration.

The therapeutic agent of endometriosis includes, in addition to danazol, well-known agents such as Buserelin, a nasal spray, which is also used as a systemic therapeutic agent.

In this circumstance, I previously proposed the use of danazol as a topical therapeutic agent (Japanese Patent Laid-Open Publication No. 221318/1989, U.S. Patent No. 4,997,653, and Asia-Oceania Journal of Obstetrics and Gynaecology, Vol. 16, No. 1, March 1990, pp. 1-12).

More particularly, I found that when danazol is topically administered, (i) the intra-uterine administration of danazol exhibits a significant effect on adenomyosis or endometriosis interna on which the oral administration of danazol have exhibited substantially no effect, (ii) the intra-vaginal administration does not inhibit ovulation and a woman who is subjected to topical treatment with danazol may be pregnant, (iii) the topical administration of danazol retains a high pregnancy rate distinguished from the case of the oral administration, (iv) the intra-vaginal administration of danazol over a long period of time successfully prevents the recurrence of endometriosis which may be often caused after the discontinuation of the oral administration of danazol, and (v) none of the side effects such as the increase of body weight, the growth of blackhead, (transient) hepatic insufficiency or the like which have often been observed in the treatment with the oral administration of danazol are accompanied with the topical administration of danazol. These knowledges indicate the incorrectness of the current theory that "complete inhibition of the endocrinological functions of the hypothalamus, pituitary and ovary" is required for danazol to exhibit an efficacy on endometriosis.

The danazol preparation for topical administration according to my prior invention comprises danazol retained on a matrix base and a release accelerating agent, if necessary. The specific matrix base includes vaginal rings and intrauterine devices.

That is, specifically, the danazol containing vaginal rings are effective on pelvic endometriosis and the danazol-containing intra-uterine devices on adenomyosis uteri, i.e. endometriosis interna. When danazol is administered topically with these devices, pituitary or ovary hormones are secreted normally without inhibition, and advantages such as the possibility of pregnancy during the insertion of the vaginal ring or after removal of the intra-uterine device, few side effects and the like are afforded.

In this connection, after the present inventor has reported the danazol preparations for topical administration, a case of the intra-vaginal administration of danazol in a dose of 500 - 600 mg once in a week was reported (The 12th Endometriosis Research Meeting, January 23-24, 1991, Kyoto Grand Hotel (Abstracts, p. 40).

Though the topical administration of danazol proposed by the present inventor is useful, it goes without saying that further modification is more preferred for such administration.

The object of the present invention is to further improve the drug form, the dosage form and/or the efficacy of the drug in the treatment of endometriosis with the parenteral non-vaginal administration of danazol.

### SUMMARY OF THE INVENTION

The therapeutic agent for endometriosis according to the present invention is a drug for parenteral non-vaginal administration to the ovary or Douglas' cul-de-sac comprising a solution of 50 mg - 20 g of danazol per 100 ml of a pharmaceutically acceptable solvent.

The present invention is advantageous for a method for the treatment of endometriosis.

According to the present invention, it is possible for a patient of endometriosis (pelvic endometriosis and adenomyosis) to be cured or remitted by topically administering a low dose of danazol (and the efficacy of the present invention is in the level same as or higher than that according to the use of the vaginal ring). Furthermore, it is possible for a patient to become pregnant even during the treatment with danazol.

It has also been found surprisingly that the dose of danazol is, e.g., about 5 mg according to the present invention which is so small an amount as eightieth to one hundredth of 400 - 800 mg (daily dose) which is a dose in the therapy by the oral dosage of commercially available danazol. The dose at 5 mg/day corresponds to a weekly dose at about 35 mg/week, which is quite a low dose even as compared with the aforementioned topical dose in the range of 500 - 600 mg/week.

The present invention also provides a danazol solution for topical dosage, and the topical injection of the danazol solution is effective against the pelvic endometriosis which is frequently developed at the sites such as Douglas' cul-de-sac and ovary and the adenomyosis which is developed within myometrium.

### DETAILED DESCRIPTION OF THE INVENTION

### Preparation for parenteral non-vaginal administration

The preparation for non-vaginal administration is a solution comprising danazol dissolved in a solvent so that the preparation has a concentration of danazol in an amount of 50 mg - 20 g, preferably 100 mg - 10 g, more preferably about 5 g, per 100 ml of the solvent.

In this connection, the phraseology "for non-vaginal administration" means that the preparation will not administered in such a way that it is administered into vagina and retained there for a while, and specifically the phraseology means the administration of the preparation to the cavity of uterus or the direct administration of the preparation into an affected part by an injection (details of which are described later).

The solvent to be used must be a pharmaceutically acceptable solvent. Thus, it should be a solvent which is not deleterious to and has little irritation on a human body, and it is preferably absorbed only slowly. Moreover, it must be a solvent in which danazol can be dissolved so that a danazol solution having a predetermined concentration of danazol is afforded. Examples of such solvents include a Lipiodol oily solution which is widely used as a contrast medium for the method for contrasting uterus and ovary, a dilute aqueous solution of ethyl alcohol, and propylene glycol, polyethylene glycol of a molecular weight of ca. 380 - ca. 420, glycerine, and the like.

### Target endometriosis

Endometriosis as a target of a therapeutic agent of endometriosis of the present invention includes both of pelvic endometriosis and adenomyosis as described above.

Pelvic endometriosis is frequently developed at the sites such as Douglas' cul-de-sac and ovary, as described above.

Adenomyosis is developed at the site of myometrium.

### Administration of the danazol preparation

The preparation for parenteral non-vaginal administration is administered parenterally and non-vaginally at one injection amount of 10 - 20 ml so that the amount of danazol is in the amount of 100 - 200 mg at the dosages of 1 - 4 times/month.

In this connection, the term "administered parenterally and non-vaginally" means specifically a method for injecting the preparation through the cervical os of the uterus, a method for injecting the preparation into an affected part on laparotomy or with a laparoscope, a method for injecting the preparation by piercing a syringe from posterior vaginal fornix through vagina into an affected part, a method for injecting the preparation into myometrium with a syringe, or the like.

Danazol injected parenterally and non-vaginally into the cavity of uterus enters into the tissue of adrenomyosis uteri to exhibit directly the effect or enters into abdominal cavity through the lumen of fallopian tube to contact directly and cure the tissue of endometriosis developed at ovary or Douglas' cul-de-sac.

As it has been confirmed by the previous investigation that the tissue of adrenomyosis uteri is in communication with the cavity of uterus, it is possible for danazol injected into the cavity of uterus to act directly upon the tissue of adrenomyosis uteri. Thus, danazol injected with pressure into uterus is injected into abdominal cavity through fallopian tube. It is necessary, however, to conduct the method for contrasting uterus and fallopian tube prior to the therapy and to confirm previously the uterine tube to be in communication by X-ray photography. As a result, in a case wherein both fallopian tubes are closed, it is possible to inject danazol intraperitoneally with a laparoscope or to inject danazol intraperitoneally by piercing a syringe into Douglas' cul-de-sac through vagina.

### Example

1) Danazol (20 mg) was dissolved in 10 ml of propylene glycol, sterilized and injected once a week into the cavity of uterus through the cervical os of the uterus. The compression and the induration of Douglas' cul-de-sac were reduced and subjective symptom was also eliminated upon the treatments of two times or more.

While it has been described above that according to the present invention, danazol exhibits effect, unexpectedly, at a lower dose as compared with the case of oral administration of danazol as well as the prior invention of the present invention and the report by the other researcher in relation to its topical administration, the following characteristics can further be mentioned as the advantages inherent to the present invention.

According to the danazol preparation according to the present invention for parenteral non-vaginal administration, the following may be mentioned.
(1) It is possible to increase sufficiently the topical concentration or the concentration at focus of danazol. Though the increase of the topical concentration has been limited in the conventional therapy by its oral dose or the therapy with the vaginal ring, the topical concentration has been increased for the first time by the therapy of the present invention, particularly the method for injecting the danazol solution.
(2) While the effect of the therapy by the oral dose of danazol has been conventionally believed to be exhibited by the lowering the secretion of hormones from hypothalamus, pituitary or ovary, I have proved that the theory is incorrect and that danazol acts directly on the cells themselves affected by endometriosis to exhibit the effect. It is as a matter of course that the therapy of the present invention by which the concentration of danazol at its acting site can be increased uppermost is most excellent in effect as well, since the functional mechanism and the acting site have been defined.
(3) The therapy by the oral administration of danazol or the therapy by the nasal administration of BUSERELIN make impossible for a patient to become pregnant, as such therapies inhibit the secretion of hormones or ovulation. In the therapy according to the present invention, a patient can become pregnant during the administration of the preparation. When the patient becomes pregnant, a large amount of progesterone is secreted and remaining endometriosis is often cured by the retention of the secretion of the progesterone over 10 months. In other words, the therapy is a novel therapy fundamentally different from the conventional therapeutic agents.
(4) Conventional therapies with drugs exhibit substantially no effect or little effect, if any, against adenomyosis uteri. According to the present invention, however, it has become possible to cure or remit it without extirpation of uterus.
   Against severe endometriosis, the therapy by administering a danazol solution into the cavity of uterus abdominal cavity exhibits a considerable effect. When the injection of the preparation does not exhibit complete cure from endometriosis, laparotomy is conducted to remove the part suffered from endometriosis as much as possible, to spread the preparation of the present invention at the end of the operation and to insert the vaginal tablet of my previous invention into vagina everyday after the operation so that the recurrence of the endometriosis can be prevented and the patient successfully becomes pregnant at a high probability.
(5) While the present invention exhibits the above-mentioned effects, it is necessary to take care of infection, as it were the side-effect, in the intra-peritoneal injection method. If the operation is conducted sterilely and combined with the oral administration of an antibiotic, such infection would be prevented. While the complication of vaginitis is observed in the treatment by inserting an intra-vaginal ring, such a side-effect would be quite seldom in the therapy of the present invention.

## Claims

1. A therapeutic agent of endometriosis for parenteral non-vaginal administration to the ovary or the Douglas' cul-de-sac comprising danazol dissolved in a pharmaceutically acceptable solvent in a concentration of 50 mg - 20 g of danazol per 100 ml of the solvent.

2. A therapeutic agent of endometriosis according to claim 1, wherein danazol is dissolved in a concentration of 100 mg - 10 g in 100 ml of the solvent.

3. The use of danazol for the manufacture of a medicament for treating endometriosis, which medicament is for parenteral and non-vaginal administration to the ovary or the Douglas' cul-de-sac in the form of a solution in a pharmaceutically acceptable solvent in a concentration of 50 mg - 20 g of danazol per 100 ml of the solvent.

## Patentansprüche

1. Therapeutisches Mittel gegen Endometriose zur parenteralen nicht-vaginalen Verabreichung an die Eierstöcke oder den Douglas-Raum, umfassend Danazol, gelöst in einem pharmazeutisch verträglichen Lösungsmittel in einer Konzentration von 50 mg bis 20 g Danazol pro 100 ml Lösungsmittel.

2. Therapeutisches Mittel gegen Endometriose gemäss Anspruch 1, in dem Danazol in einer Konzentration von 100 mg bis 10 g in 100 ml des Lösungsmittels gelöst ist.

3. Verwendung von Danazol zur Herstellung eines Medikaments zur Behandlung von Endometriose, wobei das Medikament zur parenteralen und nicht-vaginalen Verabreichung an die Eierstöcke oder den Douglas-Raum in Form einer Lösung von Danazol in einem pharmazeutisch verträglichen Lösungsmittel in einer Konzentration von 50 mg bis 20 g Danazol pro 100 ml des Lösungsmittels vorliegt.

## Revendications

1. Agent thérapeutique de l'endométriose pour administration parentérale non vaginale, dans l'ovaire ou le cul de sac de Douglas, qui comprend du danazole dissous dans un solvant acceptable d'un point de vue pharmaceutique, à une concentration de 50 mg à 20 g de danazole par 100 ml de solvant.

2. Agent thérapeutique de l'endométriose selon la revendication 1, dans lequel le danazole est dissous à une concentration de 100 mg à 10 g dans 100 ml du solvant.

3. Utilisation de danazole pour la préparation d'un médicament destiné au traitement de l'endométriose, ce médicament étant destiné à une administration parentérale et non vaginale, dans l'ovaire ou le cul de sac de Douglas, sous forme d'une solution dans un solvant acceptable d'un point de vue pharmaceutique, à une concentration de 50 mg à 20 g de danazole par 100 ml de solvant.
